# EUROPÄISCHE PATENTSCHRIFT

(11) **EP 1 417 002 B1**
(45) Veröffentlichungstag und Bekanntmachung des Hinweises auf die Patenterteilung: **12.09.2007**
(21) Anmeldenummer: 01960023.8
(22) Anmeldetag: 14.08.2001
(51) Int. Cl.: A61N 1/36, A61M 1/00, A61F 2/02

(54) **PERKUTANER ODER TRANSKUTANER ZUGANG ZUM KÖRPERINNERN**
PERCUTANEOUS OR TRANSCUTANEOUS ACCESS TO THE INTERIOR OF THE BODY
ACCES PERCUTANE ET TRANSCUTANE A L'INTERIEUR DU CORPS

(43) Veröffentlichungstag der Anmeldung: 12.05.2004
(73) Patentinhaber: PHONAK AG, 8712 Stäfa (CH)
(72) Erfinder: SCHMID, Christoph, Hans, CH-3052 Zollikofen (CH); BAECHLER, Herbert, CH-8706 Meilen (CH)
(74) Vertreter: Irniger, Ernst
(86) Internationale Anmeldenummer: PCT/CH2001/000499
(87) Internationale Veröffentlichungsnummer: WO 2001/083023

(56) Entgegenhaltungen:
- US-A- 4 004 298
- US-A- 4 352 960
- US-A- 4 726 378
- US-A- 5 144 952
- US-A- 5 507 303
- US-A- 5 949 895

## Beschreibung

Die vorliegende Erfindung betrifft eine perkutane oder transkutane Verbindungsanordnung gemäss dem Oberbegriff nach Anspruch 1, sowie Verwendungen der Anordnung, sowie ein Verfahren gemäss dem Oberbegriff nach Anspruch 12.

Aus der Literatur und aus Patenten sind verschiedenste Arten von Zugängen zum Körperinnern des Menschen bekannt. Derartige künstliche Zugänge werden beispielsweise zur Verabreichung von Medikamenten oder zur Zuführung von elektrischen Signalen oder von elektrischer Energie in den Körper benötigt.

Grundsätzlich wird zwischen perkutanen und transkutanen Zugängen unterschieden. Perkutane Zugänge führen mechanisch durch die Haut hindurch. Transkutane Zugänge weisen eigentlich keinen mechanischen Zugang auf. Häufig basieren sie auf dem Induktionsprinzip und stellen so eine elektrische Verbindung zwischen dem Innern des Körpers und seiner äusseren Umgebung her.

Bei der Ausgestaltung derartiger Zugänge spielen nebst der funktionalen Verbindung an sich, insbesondere das Einwachsverhalten an der Implantationsstelle und die Minimierung des Infektionsrisikos eine Rolle. Weiter sollten die Patienten, oder allenfalls die Pflegenden, möglichst rasch und möglichst einfach die Verbindung herstellen, respektive unterbrechen können (Bedienerfreundlichkeit).

Wo98/51367 und auch WO 99/34754 schlagen rein mechanische Steckverbindungen für das An- und Abkoppeln des Zugangs vor. Da sowohl für elektrische Zuführungen, als auch für die Verabreichung von Fluiden ein minimaler Anpressdruck an der Verbindungsstelle nötig ist, fallen die Konstruktionen relativ kompliziert aus und das an- respektive abstecken wird für den Bediener unhandlich. Im Gegensatz dazu schlägt US 5'507'303 unter anderen vor, dass der nötige Anpressdruck magnetostatisch erzeugt wird. Dabei sind sowohl im implantierten Teil, als auch im zusteckbaren Teil jeweils ein Magnet eingebaut, welche den Anpressdruck gewährleisten. Die Führung und Ausrichtung des äusseren Teils relativ zum implantierten Teil des Zugangs erfolgt jedoch mechanisch.

Aus US 5'949'895 ist eine transkutane Verbindung bekannt, welche aus einem Paar von flachen symmetrischen Spulen besteht, welche mit einem Paar von zylindersymmetrischen Permanentmagneten gegeneinander ausgerichtet werden. Auch diese letzt genannte Anordnung ist relativ kompliziert und nicht bedienerfreundlich.

Es ist daher eine Aufgabe der vorliegenden Erfindung eine perkutane oder transkutane Verbindung mit dem Körper eines Lebewesens, wie insbesondere eines Menschen vorzuschlagen, welche die eingangs erwähnten Nachteile nicht aufweist.

Die erfindungsgemäss gestellte Aufgabe wird mittels einer Verbindungsanordnung gemäss dem Wortlaut nach Anspruch 1 gelöst.

Die erfindungsgemäss vorgeschlagenen perkutanen oder transkutanen Zugänge zum Körperinnern nutzen magnetostatische Kräfte, sowohl zur Gewährleistung des nötigen Anpressdruckes, als auch zur Ausrichtung und Positionierung des äusseren Teils des Zugangs relativ zum implantierten Teil. Dadurch entsteht eine Verbindung, welche für den Bediener wesentlich einfacher zu handhaben ist. Die Positionierung kann dadurch erleichtert werden indem, dass die verwendeten Permanentmagnete asymmetrisch sind, zum Beispiel länglich sind. Nord resp. Südpole der Magnete folgen sich nicht wie in US 5'507'303 zum Körperinnern hin, sondern entlang der Körperöffnung. Mit anderen Worten verläuft der Permanentmagnet mit seinen Polen parallel zur Körperhaut.

Vorteile entstehen insbesondere dann, wenn die Verbindung zwischen dem Körperinnern und der Umgebung des Körpers eine inhärente Asymmetrie aufweist. Dies ist zum Beispiel der Fall, wenn die Zahl der individuellen Durchführungen bei einer perkutanen Verbindung nicht symmetrisch angeordnet werden kann. Ein derartiger Fall liegt ebenfalls vor, wenn zwei asymmetrische flache Spulen einer transkutanen Verbindung gegeneinander ausgerichtet werden sollten.

Die Erfindung wird nun anschliessend beispielsweise unter Bezug auf die beigefügten Figuren näher erläutert.

Dabei zeigen:
Figur 1 eine perkutane Verbindung mit zwei individuellen Durchführungen im Längsschnitt,
Figur 2 die Obendraufsicht auf die Magnete, dargestellt in Figur 1,
und Figur 3 eine schematische Darstellung einer transkutanen Verbindung mit zwei flachen physiologisch geformten Spulen im Bereich des Aussenohres.

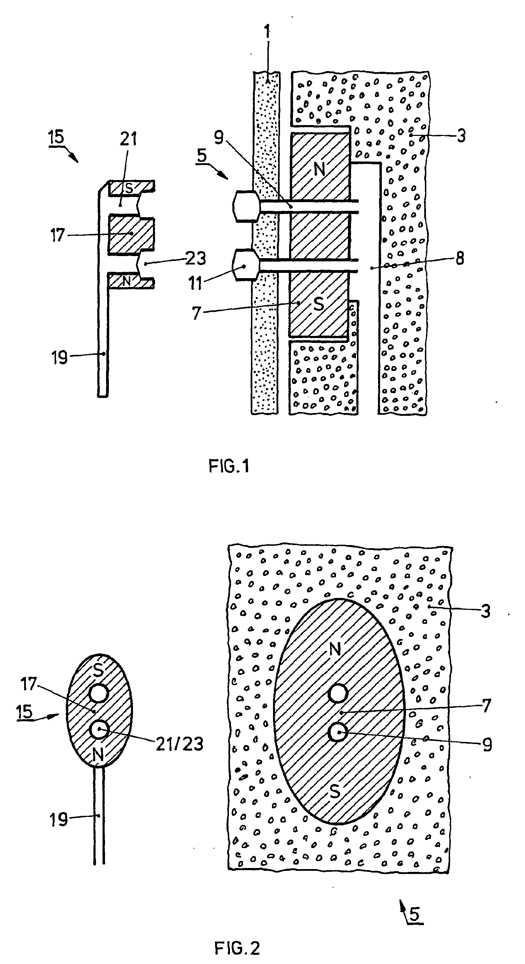

Figur 1 zeigt im Längsschnitt eine perkutane Verbindung mit zwei individuellen Durchführungen, wobei diese Verbindung zweiteilig ausgebildet ist. Das eine Teil 5 wird gebildet durch einen Permanentmagneten 7, welcher unterhalb der Oberhaut bzw. Epidermis 1 im Bereich der Lederhaut oder eines unter der Oberhaut liegenden Knochens 3 angeordnet ist. Entsprechend mit N und S bezeichnet, sind der Nord bzw. der Südpol des Permanentmagneten 7. Durch den Permanentmagneten hindurch verlaufend sind zwei Durchführungen 9 ausgebildet, welche je eine verbreiterte Öffnung 11 aufweisen, welche leicht aus der Oberhaut 1 herausragen. Selbstverständlich handelt es sich hierbei um eine beispielsweise Ausbildung der Durchführungen und es können auch nur eine oder mehrere Durchführungen vorgesehen werden und diese Durchführungen müssen nicht zwingend eine äussere vergrösserte Öffnung 11 aufweisen.

Die inneren Öffnungen der Durchführungen münden in einen mit dem Bezugszeichen 8 bezeichneten Bereich, vorgesehen für das Anordnen von Zuführ- bzw. Wegführleitungen.

Als Gegenstück dazu ist ein äusseres steckbares Teil 15 vorgesehen, welches wiederum einen Permanentmagneten 17 aufweist, umfassend einen Süd- und einen Nordpol, bezeichnet mit S und N. Von einer Zuführ- bzw. Wegführleitung 19, bei welcher es sich beispielsweise um einen flexiblen Schlauch handeln kann, verlaufen zwei Leitungen bzw. zwei Zugänge 21 durch den Permanentmagneten 17 hindurch, wiederum je aufweisend eine den Öffnungen 11 zugewandte Verbindungsöffnung 23, beispielsweise geeignet um das äussere Teil 15 auf die beiden Öffnungen 11 aufzustecken. Zusätzlich können beispielsweise Einrastelemente oder andere Halteorgane vorgesehen werden, falls die Kraft der beiden Permanentmagneten nicht ausreicht, um das äussere Teil 15 auf dem Permanentmagneten 7 zu halten.

Die Leitungen sowohl im implantierten Teil 5, wie auch im äusseren Steckteil 15, sind sowohl geeignet für das Zuführen irgendwelcher Substanzen wie Medikamente, Nährstoffe und dgl, wie auch für das Hinausführen insbesondere von Flüssigkeiten aus dem Körperinnern des Lebewesens. Letzteres ist beispielsweise der Fall bei der sog. Hämodialyse.

In Figur 2 ist einerseits der Permanentmagnet 7 in Obendraufsicht von aussen her dargestellt, wobei durch Weglassen der Darstellung der Oberhaut bzw. Epidermis 1 der Permanentmagnet 7 und die unter der Oberhaut verlaufende Lederhaut bzw. der Knochen 3 sichtbar werden. Deutlich erkennbar sind die beiden Durchführungen 9, welche senkrecht durch den Permanentmagneten 7 hindurch verlaufen. Der in Figur 2 längliche, oval ausgebildete Permanentmagnet kann selbstverständlich andersartig ausgebildet sein, wie beispielsweise rechteckig, stabförmig etc.

Analog dazu zeigt Figur 2 wiederum in Draufsicht das äussere steckbare Teil 15, primär bestehend aus dem Permanentmagneten 17 und den Zugängen bzw. Leitungen 21, welche vorgesehen sind mit den Durchführungen 9 steckbar verbunden zu werden. Auch der äussere Magnet 17 muss selbstverständlich nicht länglich oval ausgebildet sein, sondern kann, angepasst an die Form des Permanentmagneten 7, andersartig ausgebildet sein. Ja es ist gar möglich, im äusseren Teil 15 eine Spule anzuordnen zum Erzeugen eines Magnetfeldes, da der äussere Magnet 17 nicht zwingend ein Permanentmagnet zu sein hat.

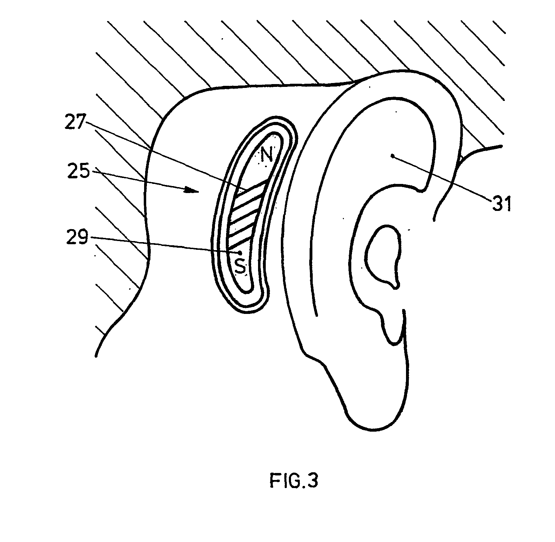

Figur 3 schliesslich zeigt eine mögliche Anwendung der erfindungsgemäss vorgeschlagenen Anordnung in Form einer transkutanen Verbindung mit zwei flachen physiologisch geformten Spulen im Bereich eines menschlichen Ohres 31.

Aufgrund der anatomischen und physiologischen Verhältnisse ist dort eine Anordnung von kreisrunden Spulen, wie sonst bei transkutanen Verbindungen oft verwendet wird, nicht geeignet. Ein Paar von ovalen, nierenförmigen oder sonst asymmetrisch geformten Spulen ist unter diesem Gesichtspunkt zu bevorzugen.

Das implantierte Teil 25 einer transkutanen Verbindung weist eine Spule 27 auf, welche zum Empfang respektive zur Aussendung von elektrischen Signalen und oder zur Übertragung von elektrischer Energie geeignet ist. Das implantierte Teil 25 weist weiter einen Permanentmagneten 29 auf, der zur Ausrichtung und Fixierung eines äusseren Teils gegenüber dem implantierten Teil dient. Diese Fixierung kann insbesondere dann notwendig sein, wenn die Spulen nicht stromdurchflossen sind. Sowohl die Spule 27, als auch der Permanentmagnet 29 sind unter der Haut implantiert und von aussen nicht sichtbar. Im Gegensatz zu perkutanen Verbindungen gibt es hier also keine mechanische Durchführung vom Körperinnern nach aussen respektive vom Äussern des Lebewesens nach innen. Figur 3 zeigt diese Anordnung, wobei durch Weglassen der Darstellung der Oberhaut über dem implantierten Teil, dieser sichtbar gemacht wurde.

Der äussere Teil (hier nicht dargestellt)der transkutanen Verbindung weist ebenfalls einerseits eine Spule zur Übertragung von Signalen und respektive oder Energie und andererseits einen Permanentmagneten zur Positionierung und Fixierung auf. Der dem implantierten Teil zugewandte Bereich des äusseren Teils der Verbindung weist Materialien und eine Oberfläche auf, welche hautverträglich und angenehm zu tragen sind. Das Paar von Permanentmagneten ist bezüglich geometrischer Form und magnetischer Stärke so ausgelegt, dass sich der äussere Teil der Verbindung optimal über dem implantierten Teil ausrichtet und positioniert, sobald dieser in die Nähe des Implantats gebracht wird. Die Ausrichtung ist dann optimal, wenn die induktive Kopplung zwischen den Spulen, bei der durch die Anatomie bestimmten geometrischen Form, maximal ist. Die Positionier- und Haltewirkung der Permanentmagneten ist auch bei dieser transkutanen Anordnung wirksam, da das magnetostatische Feld auch durch die Haut hindurch wirkt.

Die in den Figuren 1-3 dargestellten Verbindungen stellen selbstverständlich lediglich mögliche Beispiele dar und die vorliegende Erfindung ist nicht auf die dargestellten Ausführungsbeispiele eingeschränkt. So ist die Ausgestaltung des Permanentmagneten, das Anordnen des Permanentmagneten direkt in der Oberhaut oder unterhalb der Oberhaut, im Knochen, etc., die Verwendung von einer, zwei, mehreren Durchführungen oder von induktiven, kapazitiven oder sonstigen durchführungsfreien Verbindungen, etc., das Ausbilden des äusseren steckbaren Teiles etc., in den verschiedensten Variationen denkbar.

## Patentansprüche

1. Perkutane oder transkutane Verbindungsanordnung (5) zum Herstellen mindestens einer Durchführung (9) oder einer durchführungsfreien Verbindung durch die äussere Oberfläche (1, 3) eines Lebewesens, beinhaltend einen Permanentmagneten (7,29), **dadurch gekennzeichnet, dass** dieser im Bereich der äusseren Oberfläche (1, 3) mit seinen Polen und seiner magnetischen Achse weitgehend parallel zu dieser verlaufend anordenbar ist.

2. Verbindungsanordnung nach Anspruch 1, **dadurch gekennzeichnet, dass** der Permanentmagnet (7) mit den Polen weitgehend parallel zur Haut verlaufend in oder unter der Oberhaut (1) anordenbar ist und im oder am Magneten die mindestens eine, vorzugsweise mindestens zwei Durchführungen (9) vorgesehen sind.

3. Verbindungsanordnung nach einem der Ansprüche 1 oder 2, **dadurch gekennzeichnet, dass** die Anordnung ein mit den Durchführungen (9) verbindbares Teil (15) aufweist, enthaltend einen weiteren Magneten (17), wobei es sich vorzugsweise ebenfalls um einen Permanentmagneten handelt.

4. Verbindungsanordnung nach Anspruch 3, **dadurch gekennzeichnet, dass** am Permanentmagneten (7) und am weiteren Magneten (17) Positionierorgane (11,23) vorgesehen sind, um im Teil (15) vorgesehene Leitungen (19) oder Zugänge mit den Durchführungen (9) zu verbinden.

5. Verbindungsanordnung nach einem der Ansprüche 3 oder 4, **dadurch gekennzeichnet, dass** die Öffnungen(11) der Durchführungen (9) und die entsprechenden Verbindungsöffnungen (23) der Leitungen oder Zugänge (19) gleichzeitig als Positionierorgane dienen, damit das Teil positionsrichtig an den Permanentmagneten anschliessbar ist.

6. Verbindungsanordnung nach Anspruch 1, **dadurch gekennzeichnet, dass** mindestens eine durchführungsfreie Verbindung vorzugsweise, eine induktive, oder kapazitive Verbindung, zwischen dem Inneren des Lebewesens und dem Äusseren vorsehbar ist.

7. Verbindungsanordnung nach einem der Ansprüche 1 oder 5, **dadurch gekennzeichnet, dass** im Bereich des Permanentmagneten (29) mindestens eine Spule (27) anordenbar ist, vorgesehen zum Empfang respektive zur Aussendung von elektrischen bzw. elektromagnetischen Signalen und/oder zur Übertragung von elektrischer Energie.

8. Verbindungsanordnung nach einem der Ansprüche 1-7, **dadurch gekennzeichnet, dass** der Permanentmagnet (29) und vorzugsweise falls der weitere Magnet (17) länglich ausgebildet sind, vorzugsweise rechteckig oder oval.

9. Verbindungsanordnungen nach einem der Ansprüche 3-5 oder 8, **dadurch gekennzeichnet, dass** drei oder mehr Durchführungen (9) oder Leitungen oder Zugänge (19) vorgesehen sind, welche im Bezug zu einer Ebene senkrecht zu den Magneten (7,17) nicht symmetrisch angeordnet sind.

## Claims

1. Percutaneous or transcontaneous connecting arrangement (5) for providing at least one passage (9) or a passage-free connection through the outer surface (3) of a living creature, comprising one permanent magnet (7, 29), **characterized in that** said permanent magnet in the area of the outer surface (1, 3) is arranged with its poles and its magnetic axis to a large extend parallel to said external surface.

2. Connecting arrangement according to claim 1, **characterized in that** the permanent magnet (7) is adapted to be positioned with its poles to a large extend parallel to the skin in or beneath the outer skin (1) and **in that** in or at the magnet the at least one, preferably at least two passages (9) are provided.

3. Connecting arrangement according to claim 1 or 2, **characterized in that** the arrangement has one with the passages (9) connectable part (15) comprising one further magnet (17), preferably also being a permanent magnet.

4. Connecting arrangement according to claim 3, **characterized in that** at the permanent magnet (7) and at the further magnet (17) positioning elements (11, 23) are provided to connect arranged conduits (19) or access channels within the part (15) with the passages (9).

5. Connecting arrangement according to one of the claims 3 or 4, **characterized in that** the openings (11) or passages (9) and the respective connecting openings (23) of the conduits or access channels (9) serve at the same time as positioning locators for connecting the part to the permanent magnet in the correct position.

6. Connecting arrangement according to claim 1, **characterized in that** at least one passage-free connection, preferably one inductive or capacitive connection, is adapted to be arranged between the interior and the exterior of the living creature.

7. Connecting arrangement according to one of the claims 1 or 5, **characterized in that** in the area of the permanent magnet (29) at least one coil (25) is arrangable provided for receiving or sending respectively of electrical or electromagnetic signals respectively and/or for the transmission of electric energy.

8. Connection arrangement according to one of the claims 1-7, **characterized in that** the permanent magnet (29) and preferably also the further magnets (17) are designed in an elongated manner, preferably rectangular or oval.

9. Connecting arrangement according to one of the claims 3-5 or 8, **characterized in that** three or more passages (9) or conduits or access channels (19) are provided which are arranged non-symmetric with respect to a plane perpendicular to the magnets (7, 17).

## Revendications

1. Connexion (5) percutanée ou transcutanée pour établir au moins un passage (9) ou une connexion sans un tel passage à travers la surface extérieure (1, 3) d'un être vivant, comprenant un aimant permanent (7, 29), **caractérisée en ce que** l'aimant peut être disposé dans la joue de la surface extérieure (1, 3) avec les pôles et son axe magnétique s'étendant pratiquement parallèlement à ladite surface.

2. Connexion selon la revendication 1, **caractérisée en ce que** l'aimant permanent (7) avec ses pôles peut être disposé pratiquement parallèlement à la peau dans ou sous l'épiderme (1) et **en ce que** dans ou sur l'aimant sont prévus au moins une, de préférence deux passages (9).

3. Connexion selon l'une des revendications 1 ou 2, **caractérisée en ce que** la connexion comprend un élément (15) pouvant être connecté avec les passages (9) et contenant un aimant supplémentaire (17), de préférence également un aimant permanent.

4. Connexion selon la revendication 3, **caractérisée en ce que** l'aimant permanent (7) et l'aimant supplémentaire (17) possèdent des organes de positionnement (11, 23) pour connecter des conduits (19) ou accès prévus dans l'élément (15) avec ledits passages (9).

5. Connexion selon l'une des revendications 3 ou 4, **caractérisée en ce que** les ouvertures (11) des passages (9) et les ouvertures de connexion (23) correspondantes des conduits ou accès (19) servent en même temps comme organes de positionnement, pour permettre la connexion dudit élément en position correcte avec l'aimant permanent.

6. Connexion selon la revendication 1, **caractérisée en ce qu'**il est possible de prévoir au moins une connexion sans passage, de préférence une connexion inductive ou capacitive, entre l'intérieur et l'extérieur dudit être vivant.

7. Connexion selon l'une des revendications 1 ou 5, **caractérisée en ce que** l'on peut arranges au moins une bobine (27) dans la joue de l'aimant permanent (29) prévue pour la réception respectivement l'émission de signaux électriques ou électromagnétique et/ou pour la transmission d'énergie électrique.

8. Connexion selon l'une des revendications 1-7, **caractérisée en ce que** l'aimant permanent (29) et de préférence aussi l'aiment supplémentaire (17) sont de forme longitudinale, de préférence de forme rectangulaire ou ovale.

9. Connexion selon l'une des revendications 3-5 ou 8, **caractérisée en ce qu'**ils sont prévus trois ou plus des passages (9) ou conduites ou accès (19), qui par rapport à un plan perpendiculaire aux aimants (7, 17) ne sont pas disposés de manière symétrique.
